# EUROPEAN PATENT APPLICATION

(11) **EP 3 326 516 A1**
(43) Date of publication of application: **30.05.2018**
(21) Application number: 15760500.7
(22) Date of filing: 23.07.2015
(51) Int. Cl.: A61B 5/00, A61B 5/024, A61B 5/0295, A61B 5/0402, A61B 5/1455, A61B 5/0205

(54) **RECOGNITION AND TREATMENT SYSTEM FOR CARDIAC RESUSCITATION**

(71) Applicant: Betancourt Almachi, Evelyn Jacqueline, 28031 Madrid (ES); Betancourt Almachi, Boris Javier, 28031 Madrid (ES); Gómez Vicente, Rafael, Madrid (ES)
(72) Inventor: BETANCOURT ALMACHI, Evelyn Jacqueline, 28031 Madrid (ES); BETANCOURT ALMACHI, Boris Javier, 28031 Madrid (ES); BETEGON GIL, Alberto, 28031 Madrid (ES); GÓMEZ VICENTE, Rafael, Madrid (ES)
(74) Representative: Botella Reyna, Juan
(86) International application number: PCT/ES2015/070565
(87) International publication number: WO 2017/013278

(57) **Abstract**

Disclosed is a recognition and treatment system for cardiac resuscitation, which comprises: means for analyzing the cardiac rhythm in an unconscious individual by means of electrodes and a processing chip; means for indicating whether a cardiac massage is necessary; and means for measuring blood pressure using plethysmography, which can be housed in a first bracelet (1). The system also comprise means for guiding cardiac resuscitation which are housed in a second bracelet that comprises: a system of one or more accelerometers and a gyroscope, the two bracelets being autonomous and independent, and provided with: a memory; energy supply means for autonomous operation; a screen, a chronometer, and visual and audio alert means; and wireless communication means. The system can further comprise external ECG earphones that can be connected with the first bracelet.

## Description

### OBJECT OF THE INVENTION

The object of the present invention, as established in the title, is a recognition and treatment system for cardiac resuscitation used prior to the arrival of the emergency services in cases of cardiorespiratory arrest.

The present invention is characterised by the nature and functionality of each of the elements that form part of the system, in addition to the synergy arising from the joint integrated operation of the elements such that the system makes it possible to recognise whether a patient is in cardiorespiratory arrest and, if so, know whether the resuscitation operations applied are correct in terms of frequency and pressure and, furthermore, record and time the whole process carried out so that when the emergency services arrive to treat the person, they know for how long and with what frequency the person has been resuscitated.

The means used to recognise the patient's condition and supervision of the cardiac resuscitation techniques comprise electrodes, which are disposed on the affected person, a cardiac electrical signal processing chip capable of analysing the cardiac rhythm on its own and, via transmission means, connecting to an application in an electronic device, preferably a smart device, if necessary.

The system may comprise electrodes integrated in headphones such that they can perform any of the two functionalities.

The system also has integrated means to measure changes in volume in different parts of the body, particularly blood pressure based on plethysmography techniques.

It also envisages the option of including the cardiac analysis chip within the mobile device, such that a connection would be established directly between the transducers that are joined to two cables and the mobile device.

It is also composed of a support having one or more accelerometers and a gyroscope that autonomously guides the resuscitator during the cardiac massage and can connect to a smart device. Both the electrocardiographic recognition system and the cardiorespiratory arrest treatment system act autonomously, without need for a smart device, but can establish a connection by means of transmission means.

Therefore, the present invention falls under the scope of the means used in cardiopulmonary resuscitation techniques.

### BACKGROUND OF THE INVENTION

We define cardiorespiratory arrest (CRA) as the sudden, unexpected and potentially reversible interruption of spontaneous circulation and breathing, which is clinically manifested by unconsciousness, apnea and absence of pulse and which, if persisting for various minutes, irreversibly leads to biological death.

In Spain, the rate of out-of-hospital CRA due to any cause exceeds 50,000 annual cases, of which only 10% survive with minimum disability.

In Europe, there are nearly 400,000 deaths due to this cause and in the USA the figure is very similar. These figures evidence a major public health problem difficult to address.

Approximately, 80% of out-of-hospital adult CRA are of cardiac origin, basically due to coronary artery disease (atheromatosis). On many occasions (10%-26%) of CRA is the first symptom of coronary disease (sudden death) and in others exitus occurs rapidly: up to 30% of individuals who suffer myocardial infarction (sudden death) die before reaching the hospital.

The remaining 20% of CRA is of non-cardiac etiology due to internal causes (lung diseases, asphyxia, poisoning).

When cardiorespiratory arrest occurs, a cardiopulmonary resuscitation (CPR) procedure must be applied, which is the set of sequential measures aimed at reversing the cardiorespiratory arrest condition, firstly substituting the circulatory and respiratory functions and subsequently trying to restore them.

It is vital to begin the cardiopulmonary resuscitation manoeuvres at an early stage to ensure the survival of the victim. In fact, survival possibilities decrease 8% and 10% for each minute of delay and, after 10 minutes, resuscitation is normally unsuccessful and, if successful, the neurological sequelae are significant.

Basic cardiopulmonary resuscitation (CPR-B) performed by a bystander can double or triple the possibilities of survival of victims with cardiorespiratory arrest. However, bystander CPR-B is performed in a very low percentage, less than 10% of the Spanish population.

The attention protocol followed in case of cardiorespiratory arrest is composed of a series of interrelated actions on whose correct sequence the success of the resuscitation depends. These are, in particular:
a. Call: Immediate recognition of the cardiac arrest and call for help to the Emergency Medical Services (EMS).
b. Resuscitate: Early Cardiopulmonary Resuscitation (CPR) with emphasis on thoracic compressions.
c. Defibrillate: Quick application of the Automatic External Defibrillator (AED).
d. Treat: Provision of effective Advanced Vital support.
e. Monitor: Integrated post-cardiac arrest care.

The brain is the organ most sensitive to anoxia or deprivation of oxygen and will suffer irreversible damage in a short period of time: between 4 and 6 minutes without oxygen is sufficient to produce serious damage, due to which emergency measures must be applied in that brief period of time. The Spanish Cardiology Magazine highlights the need to create action strategies to reduce the response time of the resuscitation manoeuvres and palliate the population's "insufficient" knowledge in terms of basic resuscitation.

The survival chain is essential to correctly address the cardiac arrest, achieving very significant survival rates in such a dramatic situation as cardiorespiratory arrest. The basic steps of the chain are as follows:
- Fast access to an Emergency Medical System.
- Early Cardiopulmonary Resuscitation (CPR). Basic CPR precariously substitutes vital functions, but makes it possible to save precious minutes in order to apply the definitive treatment with higher possibilities of success. A multitude of studies have demonstrated how CPR survival rates decrease if basic CPR is not initiated by bystanders prior to the arrival of the professionalised team.
- Early defibrillation: is the action that most decisively determines the prognosis in VF treatment. The best results are achieved when it is possible to make the first defibrillation in less than 90 seconds or at least in less than 6 minutes.
- Advanced vital support: indicates the need to complete the stabilisation of vital functions and action on the triggering cause. The immediate results achieved with early defibrillation are consolidated when the set of advanced vital support techniques are associated in less than 10 minutes. The probabilities of survival decrease at a rate of 5.5% if none of these measures are taken.

Two serious problems arise in the unconscious individual: firstly that a lay resuscitator not trained to recognise cardiorespiratory arrest can recognise said situation in the shortest possible time. The second problem arises directly from the first: if the lay resuscitator cannot recognise that the victim is in cardiorespiratory arrest, he/she will not execute the cardiopulmonary resuscitation manoeuvres. Both healthcare professionals and lay resuscitators have trouble determining the presence or absence of adequate or normal breathing in unresponsive victims. This is because the victim may be breathing agonally, which occurs in the first few minutes after onset in 40% of cardiac arrests. Guides recommend that this assessment be made in 10 seconds, but unfortunately it is made in timeframes considered excessively long and vital in cardiorespiratory arrest or not made for fear of making an incorrect assessment. In both situations, the initiation of CPR manoeuvres is delayed excessively or they are not performed. Therefore, it is considered that the first step for initiating a cardiac massage is to recognise with absolute certainty that the victim is in cardiac arrest in the shortest possible time. This recognition is the key that opens the door to performing the cardiac massage until the arrival of the emergency services.

There are no known portable simple means that make it possible to reliably ascertain whether a cardiorespiratory arrest has occurred and, if so, whether the process of resuscitation to which a person is subjected is correct, in addition to being able to record and time the entire resuscitation process, with the object of being a source of valuable information for the emergency services when they arrive on the scene.

Therefore, the object of the present invention is to develop a system that achieves the previously indicated purposes in a simple and effective manner, developing a system such as that described below and which is set out in its essentiality in claim one.

### DESCRIPTION OF THE INVENTION

The object of the present invention is a recognition and treatment system for cardiorespiratory resuscitation used prior to the arrival of the emergency services in cases of cardiorespiratory arrest.

The system comprises:
- Means in charge of analysing the cardiac rhythm of the individual using electrodes and a cardiac electrical signal processing chip.
- Means for measuring blood pressure using the Plethysmography technique.
- Means, such as luminous, visual and/or audible signals, indicating the need or not to apply a cardiac massage.
- Means in charge of guiding the cardiac resuscitation comprising one or more accelerometers and a gyroscope.

The electrodes may be housed in a bracelet (a bracelet that analyses the cardiac rhythm) or may be housed in electrocardiographic (ECG) headphones having the necessary means for electrocardiographic analysis.

The system for recognising blood pressure by means of the Plethysmography technique supplements the analysis of an arrest or non-arrest situation, covering all the possible scenarios, thereby avoiding erroneous information.

In a possible non-limiting form of embodiment, the first means in charge of analysing the individual's cardiac rhythm consist of a bracelet having means such as:
- Means, such as a luminous, visual and/or audible signal, indicating the need to apply a cardiac massage.
- Electrodes housed in the bracelet itself on the rear side that comes into contact with the body.
- An electrocardiographic signal processing chip.
- Means for analysing blood pressure using the Plethysmography technique.

Optionally and supplementarily it may have:
- A jack for coupling ECG headphones.

In this embodiment, the means in charge of guiding the cardiac resuscitation have, as mentioned earlier, one or more accelerometers and a gyroscope, which are in charge of providing information relating to the pressure applied to the individual, the depth of the massage and frequency and, furthermore, correcting the position of the hands by analysing the angle of inclination using a gyroscope. Said information may be provided either through audible means integrated in the bracelet or visual means via a screen integrated in the bracelet.

The two bracelets, being autonomous and independent (which can be coupled to each other), are equipped with a memory in charge of storing all the data from the moment of activation, in addition to power supply means for the autonomous operation thereof, and with wireless means for connecting to any other external device (such as mobile, Tablet, computer, TV, etc.). The two bracelets would also be equipped with screens, a timer and visual and audible alert means.

When we come across an unconscious individual, we activate the recognition and treatment system such that our system is capable of detecting a rhythm susceptible of resuscitation and performing the relevant resuscitation manoueuvres on its own. It is also possible to communicate with a smart device using wireless and/or physical means such that the smart device will be in charge of recognising and treating the cardiorespiratory arrest. To this end we have a mobile application that will give us the arrest time by means of a timer, the address of the event via GPS, indications through the analysis of the information provided by our system such as, pressure, depth, frequency and correctness of the position of the hands; this application will also have visual and/or audible means to support resuscitation.

The system may be equipped with ECG headphones, which support and supplement our system, since they allow us to obtain more derivations of the electrocardiogram.

Unless specified otherwise, all the technical and scientific elements used in this specification have the meaning usually understood by the person skilled in the art to which this invention belongs. In the practice of the present invention, procedures and materials similar or equivalent to those described in the specification may be used.

Throughout the description and the claims, the word "comprises" and its variants are not intended to exclude other technical characteristics, additives, components or steps. For the experts skilled in the art, other objects, advantages and characteristics of the invention shall be partly inferred from the invention and partly from the practice of the invention.

### EXPLANATION OF THE FIGURES

In order to complement the description being made and with the object of helping to better understand the characteristics of the invention, in accordance with a preferred embodiment thereof, said description is accompanied, as an integral part thereof, by a set of drawings where, in an illustrative and non-limiting manner, the following has been represented:
Fig. 1 shows the means that form part of the system that is the object of the invention; and
Fig. 2 shows a supplementary embodiment of the invention.

### PREFERRED EMBODIMENT OF THE INVENTION

In light of the figures, following is a description of a preferred embodiment of the proposed invention.

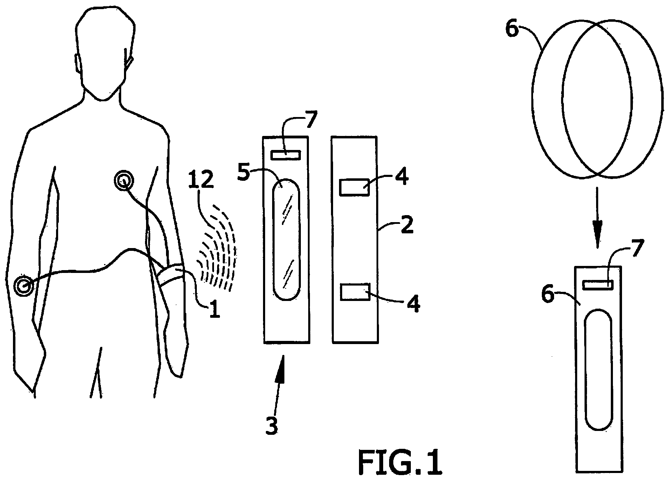

In figure 1 we can observe that the system comprises:
- A first bracelet (1) used to be placed on the unconscious patient and having an inner side (2) or that is contact with the patient, and whereon there are two integrated electrodes (4), while on the outer side (3) there are luminous means (5) indicating the need or not to perform cardiac massage.
- A second bracelet (6), a bracelet for guiding the resuscitation, which may include, inter alia, an accelerometer and a gyroscope and signal means.

In order to function autonomously and independently, the two bracelets have:
- Memory.
- Power supply means for autonomous operation.
- Screen, timer and visual and audible alert means.
- Wireless connection means (12).
- ECG headphone jack (1).

The jack (7) serves to house the ECG headphones and may be present in the first bracelet (1), in the second bracelet (2) or in both.

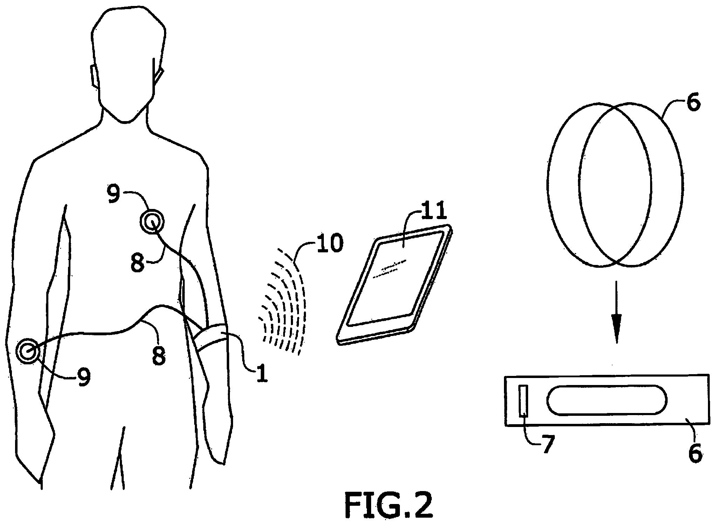

Figure 2 shows another embodiment that supplements the former in that a transmission (10) takes place from both bracelets to a smart device (11), either wirelessly or using physical connection means, such that the smart device will be in charge of recognising and treating the cardiorespiratory arrest.

Additionally, in a possible alternative embodiment the system has electrodes integrated in ECG headphones (9) connectable by means of cables (8) to the first bracelet. Said headphone electrodes (9) make it possible to embody both functionalities as desired.

Furthermore, thanks to the wireless connection means (10), it is possible to establish a connection with an external device such as a smartphone (11) with an application that, once activated, allows it to serve as a guide during the cardiac resuscitation process.

Therefore, the described system makes it possible, firstly, to recognise a cardiorespiraory arrest and, in case of detection, subject a patient in the shortest possible time to cardiorespiratory resuscitation techniques in a guided manner until the arrival of the emergency services.

Having sufficiently described the nature of the present invention, in addition to the manner in which to put it into practice, it is hereby stated that, within its essentiality, it may be put into practice in other forms of embodiment that differ in detail from that indicated by way of example and which also falls under the protection applied for, provided that it does not alter, change or modify its main principle.

## Claims

1. A recognition and treatment system for cardiac resuscitation used prior to the arrival of the emergency services in cases of cardiorespiratory arrest, **characterised in that** it comprises:
- Means in charge of analysing the cardiac rhythm of the unconscious individual using electrodes and a cardiac electrical signal processing chip;
- Means, such as luminous, visual and/or audible signals, indicating the need or not to apply a cardiac massage;
- Means for measuring blood pressure using the Plethysmography technique; and
- Means in charge of guiding the cardiac resuscitation comprising one or more accelerometers and a gyroscope.

2. The recognition and treatment system for cardiac resuscitation, according to claim 1, **characterised in that** the means in charge of analysing the cardiac rhythm, the means for measuring blood pressure and the indicator means indicating the need or not to apply a cardiac massage are housed in a first bracelet (1).

3. The recognition and treatment system for cardiac resuscitation, according to claim 2, **characterised in that** the electrodes are housed in the bracelet (1) itself, on its rear side.

4. The recognition and treatment system for cardiac resuscitation, according to claim 1, **characterised in that** the means in charge of cardiac resuscitation are housed in a second bracelet (6) and have the function of providing information relative to the pressure applied to the individual, the depth and frequency of the massage, while correcting the position of the hand by analysing the angle of inclination by means of a gyroscope.

5. The recognition and treatment system for cardiac resuscitation, according to claim 1 or 4, **characterised in that** the electrodes are electrocardiographic ECG headphones (9) connectable by means of a cable (10) to the first bracelet (1) and housable in a jack (7) of any of the bracelets (1) or (6).

6. The recognition and treatment system for cardiac resuscitation, according to claim 2 or 4, **characterised in that** the two bracelets are autonomous and independent and have a memory in charge of storing all the data from the moment of activation, in addition to power supply means for the autonomous operation thereof, and having wireless connection means (10) for connecting wirelessly to any other external device (11), and the two bracelets would also have a screen, a timer and visual and audible alert means.
